# EUROPÄISCHE PATENTSCHRIFT

(11) **EP 2 895 030 B1**
(45) Veröffentlichungstag und Bekanntmachung des Hinweises auf die Patenterteilung: **20.04.2016**
(21) Anmeldenummer: 13791725.8
(22) Anmeldetag: 13.09.2013
(51) Int. Cl.: A47C 23/28, A47C 23/24, A61G 7/002, A47C 23/18, A61G 7/015, A61G 7/057

(54) **AUFLAGEVORRICHTUNG FÜR LIEGE- ODER SITZEINRICHTUNGEN**
SUPPORT APPARATUS FOR EQUIPMENT FOR LYING OR SITTING ON
DISPOSITIF D'APPUI POUR DES INSTALLATIONS DE COUCHAGE OU D'ASSISE

(30) Priorität: 13.09.2012 AT 503902012
(43) Veröffentlichungstag der Anmeldung: 22.07.2015
(73) Patentinhaber: Wassermann, Klemens, 1210 Wien (AT)
(72) Erfinder: Wassermann, Klemens, 1210 Wien (AT)
(74) Vertreter: Sonn & Partner Patentanwälte
(86) Internationale Anmeldenummer: PCT/AT2013/050182
(87) Internationale Veröffentlichungsnummer: WO 2014/040108

(56) Entgegenhaltungen:
- EP-A2- 0 178 951
- CA-A1- 2 497 143
- DE-C- 185 713
- US-A- 3 346 891
- US-A1- 2004 133 987
- US-A1- 2011 006 582

## Beschreibung

Die Erfindung betrifft eine Auflagevorrichtung für Liege- oder Sitzeinrichtungen mit einem zumindest abschnittsweise festen Rahmen und mit verflochtenen, gespannten Gurten, die eine Auflagefläche definieren und die von der oberen Auflageseite der Auflagefläche zugänglich sind, wobei zumindest einige der Gurte mittels getrennt einstellbarer Spannvorrichtungen mit dem Rahmen verbunden sind, so dass die Vorspannung der Gurte lokal einstellbar ist.

Derartige Vorrichtungen kommen insbesondere bei Betten oder Liegen zum Einsatz, wo sich ihre Robustheit und ihr beispielsweise gegenüber reinen Metall- oder Mattenkonstruktionen höherer Komfort bewährt hat.

Die US 3,742,527 beschreibt ein Spitalbett mit einer in gegeneinander verschwenkbare Teilsegmente unterteilten Matratzenauflage mit in Längs- und Querrichtung gespannten Gurten, welche von der Unterseite zugänglich sind. Die beweglichen Teile des Rahmens der Matratzenauflage sind dabei mittels pneumatischer Antriebe einstellbar. Aufgrund der Unnachgiebigkeit der Matratzenauflage ist zwecks Liegekomfort der Einsatz einer weicheren Matratze zwischen Auflage und Benutzer notwendig, wodurch jedoch unter anderem die Luftdurchlässigkeit von der Unterseite zur Auflagefläche stark verringert und ein Durchgriff für eine Massage praktisch verhindert wird.

Die CN 201067225 Y offenbart eine Matratze, deren Kern von einem Netz verflochtener Palmenseile, welche fest in einem perforierten rechteckigen Holzrahmen angeordnet sind, gebildet ist; die Matratze ist zur Vermeidung von Deformationen des Kerns von eine feuchtigkeitsdichten Nylon-Schicht umgeben.

Die CN 2779994 Y zeigt eine Matratze mit einem durchgehend festen Holzrahmen, der mit verflochtenen Gurten umspannt ist, wobei die Spannung der Gurte nur global für die gesamte Auflagefläche nachstellbar ist.

Weiters beschreibt die DE 199 27 096 A1 eine Matratze aus einem in einem stabilen Rahmen angeordneten losen Geflecht aus Längs- und Quergurten. Die Längs- bzw. Quergurte sind dabei über an den den Gurtenden angebrachte Rollen und eine gemeinsame, in entsprechenden Rollen am Rahmen gelagerte Schnur mit dem Rahmen verbunden, so dass die Abstände zwischen den Gurtenden und dem Rahmen über die Schnur ausgeglichen werden und in Summe konstant sind. Eine lokale Einstellbarkeit der Gurtspannung ist nicht vorgesehen, sondern der Mechanismus gleicht im Gegenteil Spannungsdifferenzen zwischen den Gurten aus. Dabei kann ein loser bzw. als "schwimmend" bezeichneter Abstandhalter mit dem Gurtgeflecht verbunden sein, um den Abstand zwischen benachbarten Gurten in Bereichen von Körper-Ausbuchtungen zu limitieren.

Die DE 27 24 316 und die US 2003/0131410 A1 offenbaren Rahmen, welche mit einem Geflecht aus im Wesentlichen unelastischen Bändern bespannt sind.

Bei keiner dieser bekannten Auflagevorrichtungen kann die Spannung der Gurte lokal, d.h. für einzelne Gurte, nachgestellt werden, weshalb nachteiliger Weise keine Anpassung der Auflagefläche an die lokal veränderte Nachgiebigkeit oder an die lokal unterschiedlichen benötigten Spannungen vorgenommen werden kann.

Die AT 179048 B1 zeigt eine Kissenstütze mit einem festen Tragrahmen, in den eine Mehrzahl von Zugfedern eingespannt ist. Zwischen den Federn sind sich über die Breite und Länge der Kissenstütze erstreckende Streifen angeordnet, wobei ein Teil der Streifen nachgiebig und biegsam sowie zwischen Zugfedern eingespannt sein kann. Durch eine entsprechende Auswahl der Federn bezüglich ihrer Federstärke, ihres Durchmessers oder ihrer Spannung kann die Nachgiebigkeit der Kissenstütze bei der Herstellung bzw. beim Zusammenbau der Kissenstütze festgelegt werden.

Die CH 400 482 A betrifft eine Vorrichtung mit einem Bettrahmen, in den elastische Gurte eingespannt sind. Die Gurte sind dabei im Rahmen eingeklemmt, wobei die Gurtspannung nur aufwendig durch Lösen der Klemmverbindung, manuelles Vorspannen der Gurte und Wiederherstellen der Klemmverbindung geändert werden kann. Außerdem ist die Auswahl des Gurtmaterials bei dieser Vorrichtung durch die notwendige Dehnbarkeit der Gurte bzw. des Gurtmaterials stark eingeschränkt.

Die CA 2 497 143 A1 zeigt eine Auflagevorrichtung für einen Rollstuhl oder auch für ein Bett mit einem Rahmen über den ein Netzwerk aus Gurten gespannt ist. Beide Enden jedes Gurtes sind an der Unterseite der Auflagevorrichtung durch eine Spannvorrichtung verbunden.

Die US 2011/0006582 A1 zeigt eine aus über einen Rahmen gespannten Gurten gebildete Sitzfläche eines Rollstuhls, wobei die Gurte in verschiedenen Stellungen am Rahmen befestigt werden können.

Die US 3,346 891 A und die DE 185 713 C zeigen Betten mit einer aus verflochtenen Gurten gebildeten Auflagefläche, wobei die Gurte mit einer Feder am Rahmen befestigt sind.

### Zusammenfassung der Erfindung

Es ist Aufgabe der Erfindung, eine Auflagevorrichtung der eingangs angeführten Art zu schaffen, welche auch jenen Benutzern die Anpassung der Vorspannung an die eigenen Bedürfnisse ermöglicht, die ansonsten auf fremde Hilfe angewiesen wären, und darüber hinaus auch eine Behandlung von der Unterseite ermöglicht. Dabei soll die Nachgiebigkeit der Auflagefläche lokal einstellbar sein, so dass ein verbesserter Komfort bei der Benutzung erzielt wird. Insbesondere soll die Härte der Auflagefläche auch so einstellbar sein, dass die Verwendung einer Matratze überflüssig ist, wodurch die im Vergleich zu üblichen Matratzen hohe Luftdurchlässigkeit des Gurtnetzes dem Benützer direkt zugute kommen kann. Außerdem soll die Wahl des Gurtmaterials nicht durch Anforderungen an die Elastizität eingeschränkt werden, sondern insbesondere hinsichtlich Luftdurchlässigkeit, Gleitfähigkeit und hygienischen Anforderungen optimal auf die Verwendung als Matratze abstimmbar sein. Darüber hinaus soll eine möglichst geringe Geräuschentwicklung bei der Bewegung der Gurte gegeben sein.

Die erfindungsgemäße Auflagevorrichtung der eingangs angeführten Art löst diese Aufgabe, indem die Spannvorrichtungen zwischen Gurten und Rahmen angeordnet und die Gurte auch von der Unterseite der Auflagefläche zugänglich sind, sodass die Auflagefläche von der Unterseite zugänglich ist, indem die Spannvorrichtungen einen Antrieb zur Einstellung der Vorspannung umfassen, und indem die Antriebe der Spannvorrichtungen getrennt angesteuert bzw. ansteuerbar sind, so dass damit die Härte der Auflagefläche jederzeit lokal anpassbar ist, wobei eine lokal einstellbare Vorspannung der Gurte gemäß einem Vorspannungsprofil, z.B. automatisch, einstellbar ist. Eine geringe Geräuschentwicklung wird durch eine sehr gute Gleitfähigkeit der Gurtoberfläche erreicht. Eine lokale Einstellung der Vorspannung kann demzufolge auf einfache Weise durch Betätigung der jeweiligen Spannvorrichtung vorgenommen werden. Aufgrund der Antriebe ist eine besonders rasche Einstellung der Auflagevorrichtung möglich. Die Antriebe ermöglichen beispielsweise auch jenen Benutzern die Anpassung der Vorspannung an die eigenen Bedürfnisse, die ansonsten auf fremde Hilfe angewiesen wären. Zudem kann wegen der getrennten Ansteuerung der Antriebe eine besonders komfortable Einstellung der Auflagevorrichtung erzielt werden. In Verbindung mit einer entsprechenden Steuerung können somit verschiedene Vorspannungsprofile je nach benutzender Person und/oder momentan bevorzugter Positionierung, Körperlage oder therapeutischen Bedürfnissen eingestellt werden.

Bei der vorliegenden Auflagevorrichtung kann auch ein örtliches Nachlassen der Gurtspannung kompensiert werden, wenn das Nachlassen aufgrund starker lokaler Belastungen der Auflagefläche nur einen Teil der Gurte betrifft, ohne die Spannung der übrigen Gurte ebenfalls erhöhen zu müssen. Ebenso kann die "Härte" der Auflagefläche bereichsweise an die körperlichen Gegebenheiten des momentanen Benutzers angepasst werden, wobei beispielsweise im Fall einer Liege im Hüft-, Knie-, Fersen- und Schulterbereich eine geringere Härte eingestellt wird. Bei einem Wechsel des Benutzers oder bei veränderten Bedürfnissen können die Einstellungen jederzeit ohne großen Aufwand geändert werden, und die Auflage- bzw. Stützcharakteristik kann erneut angepasst werden. Nicht zuletzt können die Spannvorrichtungen aufgrund ihrer Anordnung zwischen Gurten und Rahmen auch Veränderungen des Rahmens antizipieren und/oder korrigieren.

Die Erfindung kann mit besonderem Vorteil bei Liegen/Betten angewandt werden, ist jedoch selbstverständlich auch bei anderen, Personen tragenden Einrichtungen, wie Fauteuils oder dergleichen Sitzmöbel, bis hin zu Kraftfahrzeug-, Bahn-, Bus- oder Flugzeug-Sitzen einsetzbar, und zwar auch für deren Rückenlehnen.

Insbesondere für die Verwendung von mehreren gleichartigen Liege- oder Sitzeinrichtungen und für einen einfachen Austausch von Teilen der erfindungsgemäßen Auflagevorrichtung ist es vorteilhaft, wenn der Rahmen mit den Gurten ein erstes Modul bildet, an dessen Unterseite eine ein zweites Modul bildende Behandlungseinrichtung angeordnet ist, welche ihrerseits auf einem von einem Unterbau gebildeten dritten Modul angeordnet ist. Somit kann das erste und im Allgemeinen leichteste Modul beispielsweise zu Reinigungszwecken von der übrigen Auflagevorrichtung getrennt werden, wodurch der Transport und die Reinigung erleichtert wird. Außerdem kann die Behandlungseinrichtung, also das zweite Modul, je nach Bedarf ausgetauscht werden, insbesondere wenn verschiedene Behandlungseinrichtungen mit verschiedenen Behandlungsfunktionen verfügbar sind, oder beispielsweise zum einfachen Ersatz defekter Behandlungseinrichtungen unter Beibehaltung des ersten und dritten Moduls und der dort vorgenommenen Einstellungen.

Bei einer effizienten und vergleichsweise kostengünstigen Variante der erfindungsgemäßen Vorrichtung sind die mit einer Spannvorrichtung verbundenen Gurte an ihrem der Spannvorrichtung gegenüberliegenden Ende fest mit dem Rahmen verbunden. Dies erfordert nur eine einseitige Einstellung der Gurt-Vorspannung, was jedoch beispielsweise in Bereichen mit relativ hohen Vorspannungen kaum nachteilig ist, da das von der Spannvorrichtung erlaubte Spiel dort ohnehin gering ist.

Wenn die Gurte an beiden Enden mit einer Spannvorrichtung verbunden sind, welche vorzugsweise dieselbe Federsteifigkeit aufweisen, kann eine Verschiebung der Gurte im mittleren Bereich der Auflage, somit gegenüber einer belasteten Stelle, zumindest teilweise vermieden werden, da die Gurte in Richtung beider Enden nachgeben. Außerdem kann in diesem Fall eine bei ansonsten gleichen Spannvorrichtungen insgesamt größere Nachgiebigkeit bzw. eine größere Bandbreite an möglichen Vorspannungseinstellungen erzielt werden.

Die feinste lokale Einstellbarkeit der Gurtvorspannung wird dann erzielt, wenn mit jeder Spannvorrichtung ein einziger Gurt verbunden ist. Insbesondere können in diesem Fall annähernd fließende Übergänge zwischen Bereichen der Auflagefläche mit höherer und geringerer Vorspannung bzw. Härte eingestellt werden, indem die dazwischen liegenden Gurte mit entsprechend abgestuften Vorspannungen eingestellt werden. Wenn mehr als ein Gurt mit einer Spannvorrichtung verbunden ist, kann die Anzahl der mit einer Spannvorrichtung verbundenen Gurte beispielsweise maximal zehn Gurte mit einer Breite von weniger als 30 mm oder maximal fünf Gurte mit einer Breite von etwa 50 mm betragen.

Es ist weiters vorteilhaft, wenn die Spannvorrichtungen eine Gleit- oder Abrollführung der Gurte aufweisen, da somit beispielsweise Schleifgeräusche der Abstützung vermieden werden können und sichergestellt werden kann, dass die Nachgiebigkeit der Gurte ausschließlich entlang des Gurtverlaufs orientiert ist. Eine zum Gurtverlauf orthogonale Belastung der Spannvorrichtung, welche deren Beschädigung oder eine Verzerrung der Härteeinstellung für die Auflagefläche zur Folge haben könnte, kann durch die zwischen Auflagefläche und Spannvorrichtung betriebsmäßig angeordnete Gleit- oder Abrollführung der Gurte vermieden werden.

Um außerdem einen unangenehmen - weil nicht von Gurten gebildeten - Randbereich der Auflagefläche so klein wie möglich zu halten, ist es günstig, wenn die Gleit- oder Abrollführung die Gurte in Richtung zur Unterseite, bevorzugt um einen Winkel von etwa 90°, umlenkt. Es können somit die Spannvorrichtungen im Wesentlichen unterhalb der Auflagefläche und platzsparend am oder im Rahmen angeordnet sein.

Insbesondere wenn die Gurte im Wesentlichen, d.h. im Rahmen der bei der gegebenen Verwendung üblichen Kräfte, unelastisch sind, ist es günstig, wenn die Spannvorrichtungen eine Federung aufweisen, wobei die von der jeweiligen Federung ausgeübte Federkraft im Wesentlichen der Vorspannung des zugehörigen Gurtes entspricht. D.h. die Spannvorrichtungen sind vorteilhafter Weise selbst elastisch und können insbesondere eine kleinere Federkonstante als die Gurte aufweisen. Zweckmäßig kann somit die Einstellung der Vorspannung über die Variation der Federauslenkung vorgenommen werden. Bei der Federung kann es sich um ein federndes Element, beispielsweise um eine Schraubenfeder, eine Bandfeder, eine Federung über elastische Werkstoffe (z.B. Gummi, Silikon) oder eine Gasdruckfeder handeln, wobei auch hier besonderes Augenmerk auf möglichst geringe Geräuschentwicklung gelegt werden sollte.

In Zusammenhang mit der Federung ist es vorteilhaft, wenn der Maximalhub der Federung bzw. die maximale Federauslenkung, bevorzugt bei einem Wert von ≤10 cm begrenzt ist. Somit kann bei typischer Weise vorübergehenden stärkeren Belastungen - beispielsweise beim Abstützen mit dem Ellbogen oder beim Aufstehen - ein übermäßiges oder unerwünscht tiefes lokales Einsinken bzw. Nachgeben der Auflagefläche vermieden werden, so dass die betreffende Tätigkeit nicht oder möglichst wenig erschwert bzw. behindert wird.

Wenn das vom Antrieb aufgebrachte Moment ausreicht, um die Vorspannung während der Verwendung der Auflagevorrichtung einzustellen, beispielsweise 1,5 Nm, abhängig von der bzw. anpassbar an die Gewichtsklasse des Benutzers und die Gurtbreite, pro Gurt beträgt, muss die Vorrichtung zur und während der Anpassung der Härte nicht entlastet bzw. verlassen werden. Das ist insbesondere bei häufigen Neueinstellungen und bei bewegungseingeschränkten Benutzern (bestimmten Krankheitsfällen) der Auflagevorrichtung von Vorteil, welche dadurch von den Vorteilen der Vorrichtung besonders profitieren können.

Eine bevorzugte Variante der Verbindung zwischen Gurt und Antrieb ist, dass der Antrieb über eine Spindel mit dem Gurt verbunden ist. Die Spindel bietet eine hohe Kraftübersetzung von einer rotierenden in eine translatorische Bewegung, was beispielsweise den Einsatz von im Vergleich zur Vorspannung relativ schwachen Elektromotoren, z.B. Schrittmotoren, ermöglicht. Zugleich ist aufgrund der Übersetzung eine hohe Präzision der Vorspannungseinstellung erzielbar.

Falls sowohl eine Federung als auch ein Antrieb eingesetzt werden, hat sich als vorteilhaft herausgestellt, wenn der Antrieb betriebsmäßig zwischen der Federung und dem Gurt angeordnet ist. Insbesondere ist bei dieser Anordnung der Antrieb gegenüber dem Rahmen gedämpft gelagert, wodurch etwaige vom Antrieb verursachte Schwingungen vom Rahmen entkoppelt und somit Geräusche reduziert sind. Davon abgesehen ist die Verbindung zwischen Antrieb und Gurt einfacher, wenn keine Federung dazwischen gelagert ist, welche nachteilig auch ein Drehmoment aufnehmen könnte.

Langfristige Veränderungen oder bestimmte zeitabhängige Funktionen können weiters dadurch realisiert werden, dass die Antriebe der Spannvorrichtungen zeitgesteuert sind und die Vorspannung der Gurte automatisch zeitabhängig veränderbar ist. Verwendungsbeispiele, die den Vorteil solcher zeitgesteuerter Veränderungen illustrieren, sind die kontinuierliche Umverteilung von belasteten Körperstellen von Patienten oder die Anpassung an die Schlafphase eines Benutzers. Mittels eines entsprechenden Steuerprogramms können ebenfalls spezielle medizinische Effekte, wie systematisch von unten (condal) oder nach oben (Richtung Herz) wandernder Druck (Wechseldruck) oder eine Unterstützung beim Abtransport von Gewebeflüssigkeit (Ödeme) erzielt werden.

Besonders zur Beobachtung, aber auch zur automatisierten Ermittlung der Vorspannungseinstellung ist es hilfreich, wenn die Spannvorrichtungen einen Kraftsensor zur Bestimmung des Auflagedrucks umfassen, wobei der Kraftsensor bevorzugt fest mit dem Gurt verbunden ist. Die Auswertung der vom Kraftsensor übermittelten Werte lässt Rückschlüsse auf die Lage, das Gewicht und mögliche Druckpunkte zu und erlaubt die automatische oder halbautomatische Anpassung des Vorspannungsprofils an die momentane Verwendung. Wenn der Kraftsensor direkt mit dem Gurt verbunden ist, kann die Kraft mit höchster Genauigkeit und Dynamik direkt erfasst werden. Für geringere Ansprüche kann auch die jeweilige Kraft indirekt über die gemessene Ansteuerleistung des Antriebes berechnet werden.

Eine besonders zuverlässige Einstellung der Vorspannprofile ist gegeben, wenn die Antriebe der Spannvorrichtungen in Abhängigkeit des vom Kraftsensor bestimmten Auflagedrucks angesteuert sind. Unter diesen Umständen kann beispielsweise automatisch auf Änderungen der Körperstellung des Benutzers reagiert werden.

Um eine hohe Sicherheit für die Benutzer auch bei Belastung einzelner Gurte zu gewährleisten, sollten die Gurte und/oder die Spannvorrichtungen eine Zugfestigkeit um mindestens einen Faktor 3 höher als die erforderliche Gurtvorspannung (abhängig von der Gewichtsklasse des Benutzers) aufweisen.

Neben der hervorragenden Luftdurchlässigkeit und der damit verbundenen natürlichen Temperierung der Auflagefläche ermöglicht die erfindungsgemäße Auflagevorrichtung aufgrund der Zugänglichkeit von der Unterseite und der geringen Dicke und Komprimierbarkeit der die Auflagefläche bildenden Gurte die Verwendung einer an der Unterseite, d.h. als Behandlungseinrichtung gemäß des oben geschilderten Modulaufbaus, angeordneten Therapievorrichtung bzw. eines Therapiegeräts, wobei das Therapiegerät bevorzugt als Massagegerät auswechselbare und/oder automatisch umschaltbare Massageköpfe und/oder Behandlungsköpfe (lokale Kühlung oder Erwärmung) umfasst.

Eine auf die anatomischen Gegebenheiten abgestimmte Massage kann dadurch ermöglicht werden, dass das Massagegerät mittels berührungsbehaftetem Abtasten und/oder berührungsloser, insbesondere kapazitiver, Messung eine automatische Lagebestimmung des auf der Auflagefläche befindlichen Benutzers durchführt. Somit können die zu massierenden Körperstellen oder -regionen unabhängig von einer absoluten Positionierung ermittelt werden. Dabei können gegebenenfalls auch die von den Kraftsensoren ermittelten Werte zur groben Orientierung bzw. Initialisierung mitverwendet werden.

Da die Temperatur an der Auflagefläche für den Komfort der Auflagevorrichtung ein zentraler Parameter ist, kann vorteilhaft je nach Bedarf am Kopf- oder Fußende des Bettes eine Heizung, beispielsweise eine elektrische Heizung oder ein Infrarotstrahler, und/oder eine Kühlung, beispielsweise ein Ventilator, ein Kühlkopf oder ein Temperierkreislauf mit einem Temperiermedium, angeordnet sein, wodurch je nach Umgebungs- und Körpertemperatur eine angenehme Temperierung der Auflagefläche vorgenommen werden kann. In diesem Zusammenhang ist es außerdem wünschenswert, bestimmte Temperaturprofile an der Auflagefläche einzubringen.

Für die Verwendung im Pflege- oder Krankenhausbereich ist es wünschenswert, wenn die Gurte aus einem hitze-, UV- und feuchtigkeitsbeständigen Material hergestellt sowie thermisch und chemisch desinfizierbar sind. Eine derartig pflegeleichte und beispielsweise einfach mit einem Heißwasser-Hochdruckreiniger desinfizierbare Vorrichtung kann ohne Einschränkung auch unter hygienisch anspruchsvollen Bedingungen angeboten werden, da somit keine zwischen Auflagefläche und Benutzer eingezogene dichte Schutzschicht notwendig ist. Derartige Schutzschichten, wie sie beispielsweise zum Schutz von Matratzen gebräuchlich und notwendig sind, würden die Luft- und Temperaturdurchlässigkeit der Vorrichtung sowie die Therapie-/Massagemöglichkeiten stark beeinträchtigen.

Um die Geräuschentwicklung sowie etwaige ungewollte Verschiebungen beim Einstellen der Spannvorrichtungen weitgehend zu vermeiden, ist es vorteilhaft, wenn die Gurte eine reibungsarme bzw. gleitfähige Oberfläche aufweisen, wobei der Reibungswiderstand zwischen den Gurten so gering ist, dass er gegenüber der Vorspannung vernachlässigbar ist, wobei auch Geräusche bei der Verschiebung der Gurte minimiert werden.

Bei der Verwendung für unbewegliche oder schwierig umzubettende Patienten im Krankenhaus- und/oder Pflegebereich ist es weiters nützlich, wenn die Auflagefläche eine mit einer Matratze vergleichbare oder geringere Absorptionsfähigkeit für Röntgenstrahlen aufweist, da somit diverse Diagnoseverfahren direkt auf der Auflagevorrichtung durchgeführt werden können, was insbesondere durch die Zugänglichkeit der Auflagefläche von der Unterseite begünstigt wird.

Die bisher genannten Vorteile können beispielsweise dann erzielt werden, wenn die Gurte im Wesentlichen aus Polyestergeflecht-, Kunstseide-, Goretex- oder Aramid-Bändern, alternativ aus Hybridgeweben (z.B. verstärkt mit Carbonfasern) bestehen. Zur Erhöhung der Gleitfähigkeit können die Gurte zusätzlich eine Beschichtung, insbesondere eine TeflonBeschichtung, aufweisen.

Wenn der Rahmen in Längsrichtung in bevorzugt mindestens zwei, insbesondere vier, durch Gelenke miteinander verbundene Teilsegmente unterteilt ist, wobei die Gelenksachsen quer zur Längsrichtung des Rahmens angeordnet sind, kann die Auflagevorrichtung eine Vielzahl von Körperhaltungen unterstützen. Die Teilsegmente können parallel ausgerichtet oder mechanisch bzw. über Hilfsantriebe, beispielsweise für Krankenbetten häufig verwendete, industriell verfügbare Linearantriebe, sogenannte Hubsäulen, in der Winkellage verstellt werden. Beispielsweise kann dieselbe Vorrichtung je nach Anordnung der Teilsegmente entweder als Liege- oder als Sitzeinrichtung, zum Anheben des Kopfteils oder im Kniebereich, verwendet werden.

Als Unterstützung der Einstellung der Winkellage der Teilsegmente können mit dem Rahmen Antriebe verbunden sein, die eine bestimmte Einstellung bewirken bzw. eine Bewegung der Teilsegmente unterstützen. Die Steuerung der Lage der einzelnen Segmente der Auflage und die Vorgabe verschiedener therapeutischer Massageprogramme bzw. Kühl- oder Wärmemaßnahmen erfolgt über einen Bediencomputer (Human Machine Interface, kurz HMI) oder ferngesteuert. Dabei sind verschiedene Sicherheitsstufen für die Parameter-Veränderung oder -Eingabe vorgesehen. Therapeuten und Mediziner haben, mit einem speziellen Passwort, Zugang zur höchsten Sicherheitsstufe, mit der Möglichkeit alle verfügbaren Parameter und Programme für den Benutzer vorzugeben. Medizinisches Pflegepersonal hat Zugang zu einem reduzierten, ebenfalls über ein spezielles Passwort geschützten, Parameterbereich bzw. kann vordefinierte therapeutische Behandlungs-Programme zeitgesteuert zur Anwendung bringen. Von höheren Sicherheitsstufen freigeschaltete Funktionen können ohne Passwort vom Benützer jederzeit aktiviert werden. Bei Verwendung eines Bettes für mehrere Benützer können im HMI benützerspezifische Kenndaten, beispielsweise das Steifigkeitsprofil der Gurte, abgespeichert und abgerufen werden.

Besonders in öffentlich zugänglichen Einrichtungen, wie Krankenhäusern, Pflegeanstalten oder auch Hotels, wenn eine Auflagevorrichtung an verschiedenen Orten und für verschiedene Benutzer zum Einsatz kommt, ist es günstig, wenn der Unterbau gebremste Rollen zum Verschieben der Vorrichtung sowie einen vorzugsweise elektrischen, alternativ hydraulischen oder pneumatischen Hilfsantrieb zum Heben und Senken bzw. Schrägstellen der Auflagevorrichtung, insbesondere einzelner Teilsegmente des Rahmens, aufweist.

Ebenfalls, insbesondere bei Verwendung mehrerer erfindungsgemäßer Auflagevorrichtungen in einem einzelnen Raum, ist es vorteilhaft, wenn die Geräusch- bzw. Lärmentwicklung bei allen Verwendungen auf ein Minimum reduziert ist. Dabei ist besonderes Augenmerk auf die Abrollführungen, die Federungen und die Antriebe zu legen, aber auch die Reibung zwischen den Gurten sollte möglichst gering gehalten werden. Bei Verwendung einer Massageeinrichtung oder einer Kühl- oder Heizvorrichtung ist auch hier eine geringe Geräuschentwicklung wünschenswert und typischerweise durch geeignete Antriebe, wie Direktantriebe mit Zahnriemen, erzielbar.

### Kurze Beschreibung der Zeichnungen

Die Erfindung wird nachfolgend anhand von besonders bevorzugten Ausführungsbeispielen, auf die sie jedoch nicht beschränkt sein soll, und unter Bezugnahme auf die Zeichnung noch weiter erläutert. In der Zeichnung zeigen dabei im Einzelnen:
Fig. 1 eine schematische Draufsicht auf eine erfindungsgemäße Auflagevorrichtung;
Fig. 2 eine schematische Detailansicht einer Abrollführung gemäß dem Ausschnitt II in Fig. 1;
Fig. 3 in schematischer Draufsicht in der Teil-Fig. 3A einseitig mit Spannvorrichtungen verbundene Gurten und in Fig. 3B beidseitig mit Spannvorrichtungen verbundene Gurten;
Fig. 4 einen schematischen vertikalen Schnitt durch eine Spannvorrichtung gemäß Fig. 2;
Fig. 5 schematisch eine elektronische Regelschaltung zur Anlegung eines Vorspannungsprofils;
Fig. 6A eine schaubildliche Ansicht einer Auflagevorrichtung (ohne Gurtgeflecht) aus drei Modulen mit einer Massageeinrichtung;
Fig. 6B eine schematische Explosionsdarstellung der drei Module gemäß Fig. 6A;
Fig. 6C eine perspektivische Ansicht eines Massagekopfs der Massageeinrichtung gemäß Fig. 6A;
Fig. 6D eine perspektivische Ansicht eines lokal wirksamen Wärme- oder Kühlkopfes gemäß Fig. 6A; und
Fig. 7 mit den Teilfiguren A bis F schematisch sechs Stellungen der Auflagevorrichtung gemäß Fig. 6A.

### Detaillierte Beschreibung der Zeichnungen

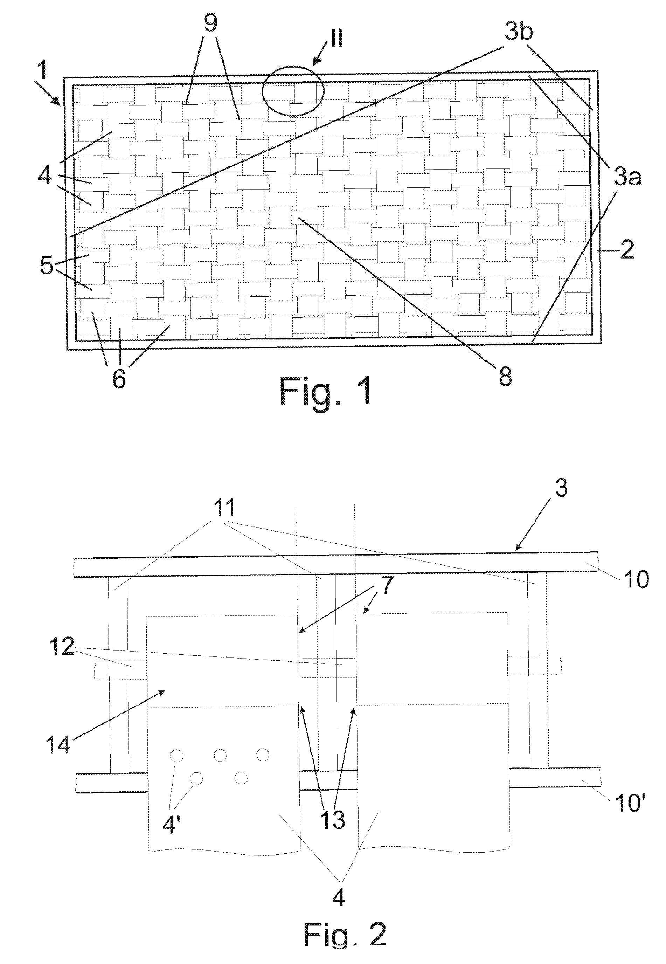

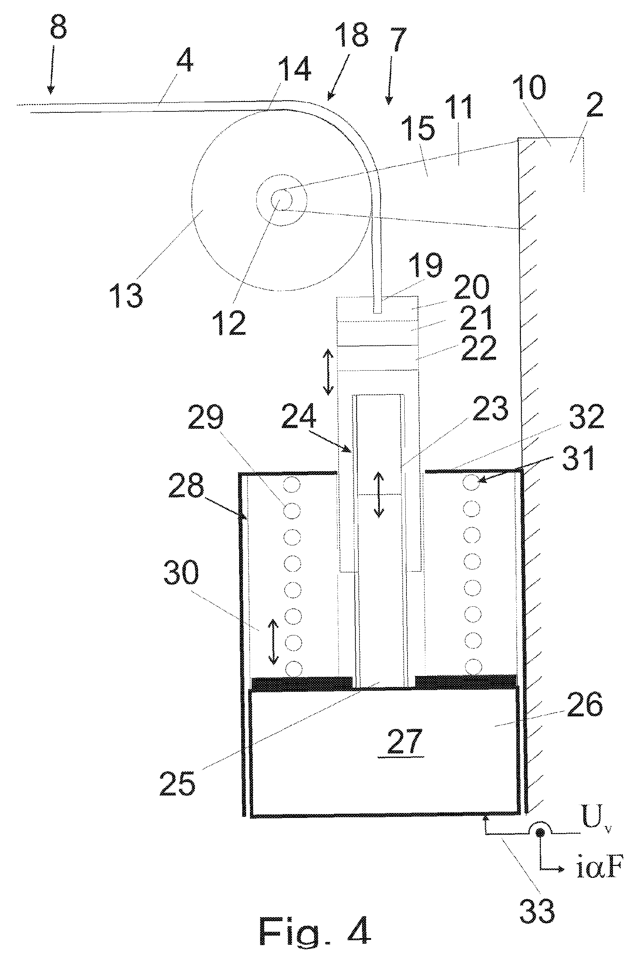

In Fig. 1 ist in einer schematischen Draufsicht eine Auflagevorrichtung 1 für eine Liegeeinrichtung, insbesondere ein Bett, gezeigt. Die Auflagevorrichtung 1 weist einen in Draufsicht rechteckigen Rahmen 2 auf. Im Rahmen 2 sind zwischen einander gegenüberliegenden Rahmenelementen 3a, 3b Gurte 4 gespannt. Die Gurte 4 verlaufen in zwei Gruppen parallel entweder in Längsrichtung des Rahmens 2 oder quer dazu und sind mit gleichen Abständen angeordnet. Der Abstand, d.h. der freie Raum 9 zwischen den Gurten 4, sollte 10 mm nicht überschreiten, um ein Durchbrechen, beispielsweise mit dem Ellbogen beim Abstützen zu unterbinden. In einer bevorzugten Ausführung beträgt der Gurtabstand etwa 5 mm. Um ein Verrutschen der Gurte 4 quer zur Spannungsrichtung zu vermeiden, sind die Gurte 4 verflochten, wobei beispielsweise ein Längsgurt 5 abwechselnd über und unter aufeinanderfolgenden Quergurten 6 angeordnet ist. Abhängig von der gewählten Gurtbreite können aber auch alternative Flechtkombinationen (z.B. jeweils zwei Längs- und/oder Quergurte) gewählt werden. Die Breite paralleler Gurte 4 ist im Wesentlichen konstant, wobei im gezeigten Beispiel die Längsgurte 5 schmäler sind als die Quergurte 6. Eine Gurtbreite von mindestens 5 mm, bevorzugt zwischen 30 und 200 mm, insbesondere 50 mm, stellt einen geeigneten Kompromiss zwischen lokaler Einstellbarkeit (Komfort), Festigkeit und Wirtschaftlichkeit dar. Schmälere Gurte 4 ermöglichen eine feinere Einstellung der lokalen Steifigkeit und damit einen höheren Komfort durch eine gleichmäßigere Druckverteilung. Sie sind besonders in Bereichen hoher Auflagedrücke (Sakral-, Knie-, Schulter- und Fersenbereich) vorteilhaft. Breitere Gurte 4 erfordern einen weniger hohen technischen und damit finanziellen Aufwand. An beiden Enden sind die Gurte 4 mit den Rahmenelementen 3a, 3b verbunden, wobei die Verbindung zumindest an einem Ende und zumindest an einer Querseite des Rahmens 2 über Spannvorrichtungen 7 (vgl. Fig. 4) hergestellt ist. Die vom Rahmen 2 eingeschlossene Fläche ist von dem Gurtgeflecht abgedeckt, so dass die Gurte 4 eine Auflagefläche 8 bilden. Die zwischen den Gurten verbleibenden rechteckigen Öffnungen 9 in der Auflagefläche 8 haben den Abständen zwischen den Gurten 4 entsprechende Kantenlängen und bestimmen eine relativ hohe Luftdurchlässigkeit der Vorrichtung. Zusätzlich können in den Gurten 4 Perforationen 4' (vgl. Fig. 2) (Lochraster) vorgesehen sein, um die Belüftung der Auflagefläche 8 von der Unterseite her weiter zu verbessern.

Was das Material und die Struktur der Gurte 4 betrifft, so müssen diese nicht nur die mechanischen Anforderungen, beispielsweise eine bestimmte Zugfestigkeit, erfüllen, sondern oft zusätzlichen Anforderungen hinsichtlich Hygiene und Reinigungsmöglichkeiten genügen. Insbesondere ist es wünschenswert, dass nicht - wie bei gewöhnlichen Matratzen oder Schaumstoffeinlagen - mit Plastikeinlagen gearbeitet werden muss, welche den Komfort stark mindern würden. Wenn die Gurte 4 beispielsweise aus Polyestergeflecht-, Kunstseide-, Goretex- oder Aramid-Bändern, alternativ aus Hybridgeweben (z.B. verstärkt mit Carbonfasern, ohne oder mit Beschichtung zur Erhöhung der Gleitfähigkeit, beispielsweise mit Teflon) bestehen, kann die Auflagefläche 8 direkt mit einem Hochdruckreiniger gereinigt und entsprechend den hygienischen Anforderungen auf die Verwendung vorbereitet, insbesondere desinfiziert, werden.

Fig. 2 zeigt zwei Spannvorrichtungen 7 gemäß dem Detail II in Fig. 1, wobei ersichtlich ist, dass das Rahmenelement 3a bzw. 3b aus zwei parallelen Längsstreben 10, 10' und dazwischen quer angeordneten Rollenabstützungen 11 aufgebaut ist. Die Gurtbreite und der Gurtabstand sind hier nicht maßstabsgetreu abgebildet, sondern würden bevorzugt 50 mm respektive 5 mm betragen. In den Rollenabstützungen 11 sind parallel zu den Längsstreben 10 Rollenachsen 12 angeordnet, welche als Abstützung der eine Abrollführung bildenden Rollen 13 dienen. Der Abstand zwischen den Längsstreben 10, 10' ist größer als der Durchmesser der Rollen 13, so dass der Abstand zwischen der Rolle 13 und der außenseitigen Längsstrebe 10 zumindest der Stärke des Gurts 4 entspricht. Vorzugsweise ist die Höhe der innenseitigen Längsstrebe 10' so gewählt, dass der Scheitel 14 der Rolle 13 über die innenseitige Längsstrebe 10' ragt, damit der Gurt 4, ohne diese zu berühren bzw. auf ihr zu schleifen, darüber gespannt ist. Alternativ kann auf eine innenseitige Längsstrebe 10' auch vollständig verzichtet werden (vgl. Fig. 4), wobei die Rollenachsen 12 und damit die Rollen 13 dann beispielsweise von frei auskragenden Konsolen 15 als Rollenabstützungen 11 getragen werden.

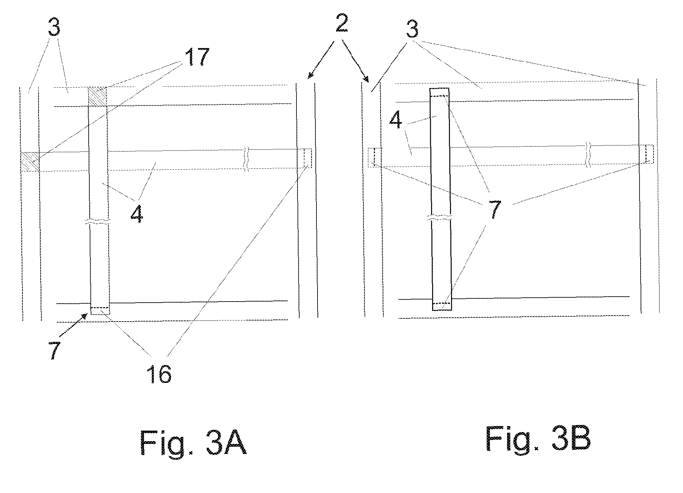

Wie in Fig. 3A gezeigt, können die Gurte 4 nur an einem Ende 16 mit einer Spannvorrichtung 7 verbunden sein. Das der Spannvorrichtung 7 gegenüberliegenden Ende 17 ist dann beispielsweise fest mit dem Rahmen 2 verbunden. Dabei können die Spannvorrichtungen 7 aller Gurte 4 an einem Rahmenelement 3a, 3b angeordnet sein, oder die Spannvorrichtungen 7 aufeinanderfolgender paralleler Gurte 4 sind abwechselnd einmal an dem einen und dann am gegenüberliegenden Rahmenelement 3 angeordnet. Der geringste Herstellungsaufwand wird dann erzielt, wenn an nur zwei zueinander rechtwinkeligen Rahmenelementen 3 (einem Querelement und einem Längselement) sämtliche Spannvorrichtungen 7 angeordnet sind.

Einen größeren Komfort bieten allerdings beidseitig mit den Gurten 4 verbundene Spannvorrichtungen 7, wie in Fig. 3B gezeigt. Aufgrund der geringeren Reibung zwischen den Gurten 4 ermöglicht diese Variante auch eine Reduktion der Geräuschentwicklung bei der Verwendung.

In Fig. 4 ist schematisch der Aufbau einer bevorzugten Ausführungsform einer Spannvorrichtung 7 im Detail dargestellt. Wie bereits in Zusammenhang mit Fig. 2 erläutert wird der Gurt 4 über eine Abrollführung 18 mit einer Rolle 13 geführt. Wie hier im Detail ersichtlich, führt die Abrollführung 18 den Gurt 4 ausgehend von einer horizontalen Auflagefläche 8 entlang eines Viertelsegments der Rolle 13 um 90° in eine Vertikale. Die Rolle 13 der Abrollführung 18 ist dabei um eine Rollenachse 12 drehbar gelagert, wobei die Rollenachse 12 in der als Rollenabstützung 11 vorgesehenen Konsole 15 angeordnet ist, welche ihrerseits mit dem außenseitigen Rahmenelement 10 verbunden ist. Das vertikal nach unten weisende Gurtende 19 ist in einer Klemme 20 angeordnet und gehalten. Die Klemme 20 ist über ein Lager 21 mit einem Kraftsensor 22, z.B. mit einem DMS-Element, verbunden. An der dem Lager 21 gegenüberliegenden Seite des Kraftsensors 22 ist dieser auf eine ein Feingewinde 23 aufweisende Gewindespindel 24 angekoppelt und gegen Verdrehen gesichert. Der Kraftsensor 22 misst somit den Zug direkt zwischen der Spindel 24 und dem Gurt 4. Die das Feingewinde 23 aufweisende Spindel 24 ist mit der Welle 25 eines Antriebs 26, insbesondere eines Elektromotors 27 verbunden. Der Antrieb 26 selbst ist schließlich über eine Federung 28 mit dem Rahmen 2 der Auflagevorrichtung verbunden. Die Federung 28 ist von einer Druckfeder 29 und/oder einem zusätzlich federnden Füllstoff 30, beispielsweise Gummi oder Silikon, gebildet und wird durch die Gurtvorspannung komprimiert. Im gezeigten Beispiel wird die Gurtspannung durch die mit Hilfe des Antriebs 26 einstellbare Position der Welle 25 in der Gewindespindel 24 vorgegeben. Die Abstützung der Federung 28 erfolgt über die horizontale Angriffsfläche 32, die fest mit dem Rahmenelement 10 verbunden ist. Als alternative Ausführung könnte auch ein gemeinsamer Antrieb für eine Rahmenseite für ein Rahmenelement 3a bzw. 3b, gewählt werden, wobei die Verstellung der jeweiligen Gurtvorspannung durch eine gezielte Ankopplung des Antriebes (z.B. verschiebbares Zahnrad) an die Gewindespindel 24 erfolgt.

Der Maximalhub der Federung 28 ist durch die maximale Komprimierbarkeit der Federelemente 29 bzw. 30 definiert. Bei - praktisch unmöglicher - vollständiger Kompression der Federung 28 würde der Antrieb 26 maximal bis zur Anlage an die Angriffsfläche 32 nach oben nachgeben. Die Einstellung der mechanischen Gurtvorspannung erfolgt über die Versorgungsleitung 33 des Antriebs 26, wobei ein entsprechend gepolter Strom oder bei einem Schrittmotor eine Impulsfolge über einen bestimmten Zeitraum die gewünschte Spindeldrehung in die eine oder andere Richtung bewirkt. Wenn der Gurt 4 nicht selbst als Federung wirkt, führt die von der Spindel 24 verursachte translatorische Bewegung zu einer Kompression oder Dekompression der Feder 29 und/oder des Federfüllstoffs 30 und dadurch zu einer Steigerung oder Absenkung der Vorspannung des Gurts 4. Wegen der Umlenkung des Gurts 4 wirkt ohne Belastung der Auflagefläche 8 die der Vorspannung entsprechende Kraft nicht nur horizontal zwischen den gegenüberliegenden Rahmenelementen 3a bzw. 3b, sondern auch vertikal zwischen der Abrollführung 18 und der Angriffsfläche 32 der Federung 28.

Der Hub der Gewindespindel 24 ist zweckmäßig höchstens gleich groß wie der Maximalhub der Federung 28, wobei die Länge des vertikalen Gurtabschnitts insbesondere bei geringen Vorspannungen so gewählt sein muss, dass es bei Belastung der Auflagefläche nicht zu einer Kollision der Gurtklemmung 20 und der Rolle 13 kommt.

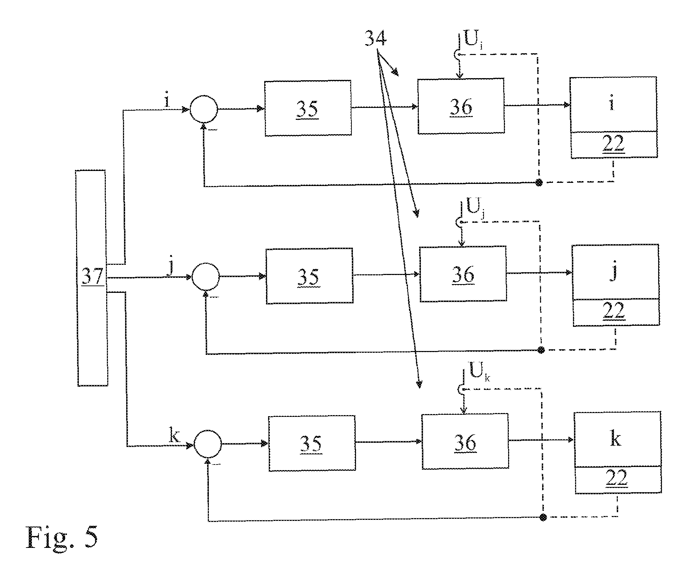

Die in Fig. 4 dargestellte, im Wesentlichen unnachgiebige Verbindung zwischen Antrieb 26 und Kraftsensor 22, insbesondere ohne zwischengeordnete Federung, erzielt eine unmittelbare und störungsfreie mechanische Rückkopplung vom Antrieb 26 auf den Kraftsensor 22 in einer Regelungsschleife 34, wie beispielsweise in Fig. 5 gezeigt. Hier sind schematisch Regelungsschleifen 34 für drei unabhängige Spannvorrichtungen 7 dargestellt, die ihre jeweiligen Sollwerte, auf welche die Gurtvorspannung geregelt wird, aus einem gemeinsamen Vorspannungsprofil beziehen. Die einzelnen Regelungsschleifen 34 weisen neben den Kraftmessungen bzw. Kraftsensoren 22 auch Regler 35 und Aktoren 36 (entsprechend den Antrieben 26) auf. In der Praxis wird ein wählbares Vorspannungsprofil über eine zentrale, programmtechnische Sollwertvorgabe für die Regler mit der Einkopplung 37 vom Bediencomputer (HMI) 43 vorgegeben. Die Regler 35 liefern das erforderliche Ansteuersignal für die Aktoren 36 basierend auf der Istwerterfassung durch die Kraftmessung 22 und/oder die Strommessung (Uᵢ, Uⱼ, Uₖ, etc.).

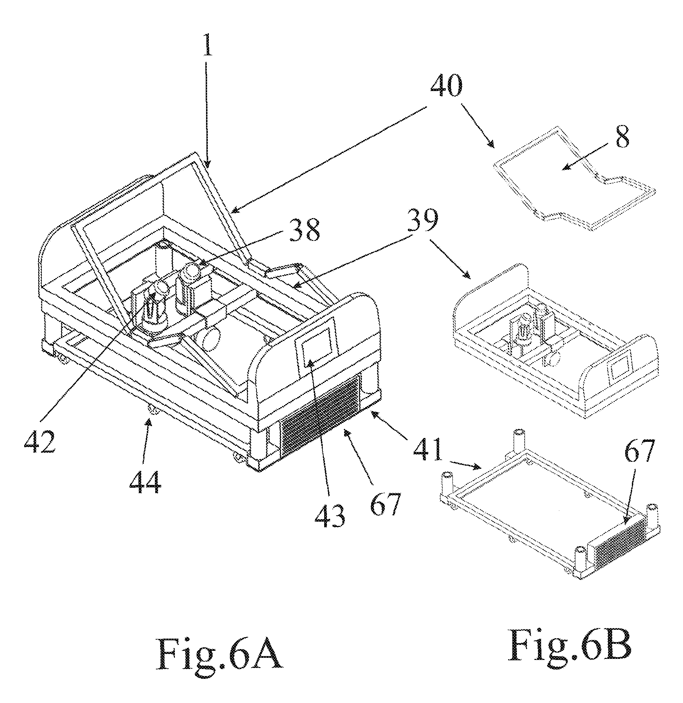

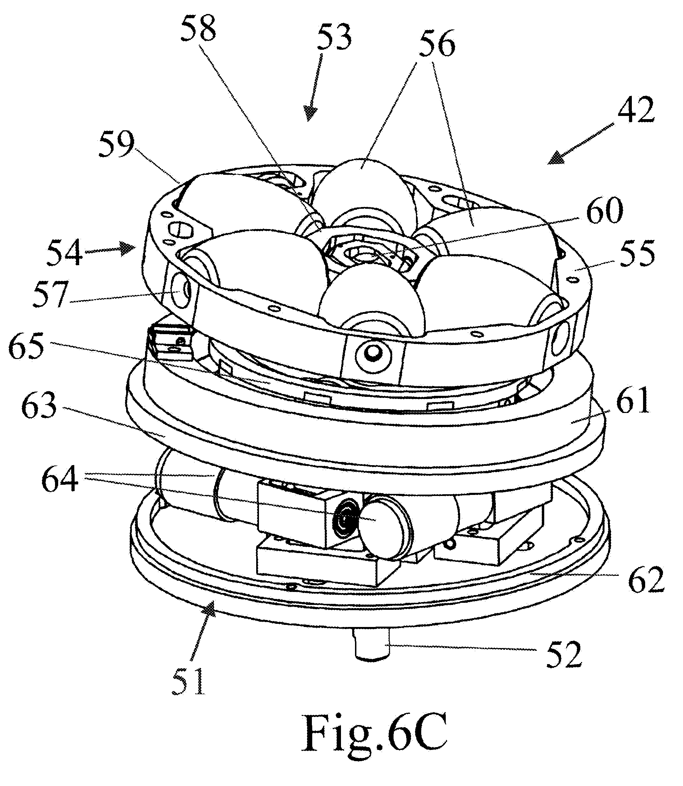

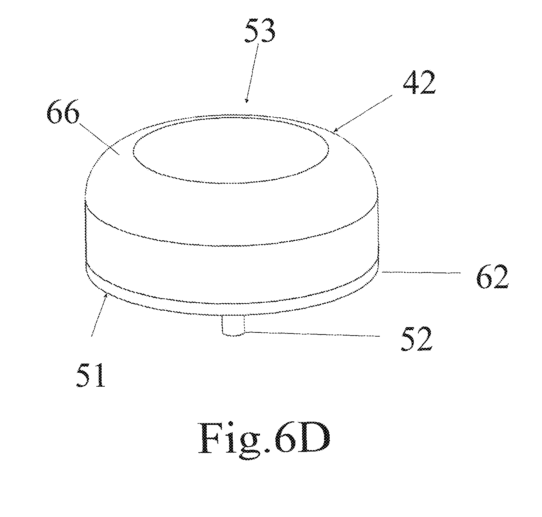

Ein - neben dem Komfortgewinn - zentraler Vorteil der vorliegenden Auflagevorrichtung 1 ist die Zugänglichkeit der Auflagefläche 8 auch von der Unterseite und die dadurch ermöglichten Anwendungsszenarien. Der Vielseitigkeit an Einsatzmöglichkeiten kommt der in Fig. 6A und 6B beispielhaft gezeigte Modulaufbau der Auflagevorrichtung 1 besonders entgegen. Auch wenn hier lediglich eine Massageeinrichtung 38 als Behandlungseinrichtung 39 dargestellt ist, ist es dem Fachmann verständlich, dass zwischen dem ersten Modul 40 (in Fig. 6 nur schematisch, ohne Gurte, gezeigt) mit der Auflagefläche 8 und dem dritten Modul 41, welches einen universellen Unterbau bildet, verschiedenste Behandlungseinrichtungen 39 Verwendung finden können. Insbesondere sei hier auf den Einsatz eines "Wärmetools" oder eines "Kühlungstools" (vgl. Fig. 6D), beispielsweise eines Infrarotstrahlers bzw. einer elektrischen Widerstandsheizung oder eines vorgekühlten Kältekopfs - wahlweise einsetzbar statt eines Massagekopfes 42 - in der Massageeinrichtung 38 zum lokalen Erwärmen oder Abkühlen der Auflagefläche 8 bzw. von Körperregionen des Benutzers hingewiesen. Zur globalen Erwärmung oder Abkühlung der Auflagefläche 8 kann das dritte Modul 41, beispielsweise am Fußende, eine Heizung (z.B. elektrische Heizung oder Infrarotstrahler) und/oder ein Kühlaggregat 67 (z.B. mit Peltier Elementen oder speziellen Kühlmedien und Kühlkreisläufen) samt Ventilation mit regelbarer Drehzahl aufweisen.

Aufgrund der geringen Dicke der Auflage bzw. der Gurte 4 kann bei Verwendung mit einer Massageeinrichtung 38 ein guter Durchgriff erzielt werden, was insbesondere lokale Behandlungen begünstigt. Es können folglich nach therapeutischen Grundsätzen gesteuerte Massagebewegungen für Nacken, Rücken und Beinbereich, wie Effleurage, Walken, Klopfen und Vibrieren, von der Unterseite der Auflagevorrichtung 1 durchgeführt werden. Zur Durchführung der verschiedenen Techniken weist die Massageeinrichtung 38 Massageköpfe 42 (vgl. Fig. 6C) auf, die beispielsweise jeweils mit sechs Freiheitsgraden (drei Rotationsachsen und drei Translationsrichtungen) bewegt werden können. Außerdem können verschiedene Massageköpfe 42 vorgesehen sein, die manuell oder automatisiert gewählt und eingesetzt werden. Mögliche Anwendungen sind beispielsweise Lymphdrainagen, (Mikro-)Stimulationen zur Vermeidung gesundheitlicher Schäden durch lange Immobilisation (z.B. Dekubitus) und Verlust des Selbstgefühls für einzelne Körperteile (insbesondere Schulter, Sakralregion und Fersen), psychische Entspannung, eine Stärkung des Immunsystems, Verbesserung der Mikrozirkulation und die Prävention von Druckgeschwüren. Die automatisierte Durchführung entsprechender Therapien ermöglicht nicht nur Entlastungen des Pflegepersonals, sondern begünstigt auch eine schnelle Genesung und somit kürzeren Aufenthalt speziell in Krankenanstalten. Zur Vorbereitung des jeweiligen Massageprogramms wird die aktuelle Lage der zu massierenden Person zu Beginn aber auch während der Massage durch eine flächige, sehr sanfte Abtastung, entsprechend der Effleurage, mit der Massageeinrichtung 38 oder berührungslos (z.B. mit induktiven, optischen oder Infrarot-, bevorzugt kapazitiven Sensoren) bestimmt.

In Fig. 6C ist ein Massagekopf 42 im Detail gezeigt. Der Massagekopf 42 ist über die Unterseite 51 und über eine Drehachse 52 mit der Massageeinrichtung 38 (vgl. Fig. 6A) verbunden. An der Oberseite 53 weist der Massagekopf 42 ein kreisförmiges Massagewerkzeug 54 mit einem Lagerkranz 55 und sechs darin drehbar gelagerten bauchigen Massagewalzen 56 auf. Die Achsen 57 der Massagewalzen 56 bilden die Speichen des Lagerkranzes 55 und sind radial zwischen einer zentralen Nabe 58 und einem Außenring 59 angeordnet. Das Massagewerkzeug 54 ist dabei nicht nur um die Drehachse 52 drehbar, sondern dank einer kardanischen Aufhängung 60 der Nabe 58 auch gegenüber dem übrigen Massagekopf 42 in alle Richtungen schwenkbar. Unterhalb des Massagewerkzeugs 54 weist der Massagekopf 42 eine rotierende Führung 61 des Lagerkranzes 55 auf. Zwischen einem Begrenzungsring 63 und dem kreisförmigen Boden 62 des Massagekopfs 42 ist eine elastische Abdichtung (nicht eingezeichnet) vorgesehen. Eine elastische Dichtung ist ebenfalls für den oberen Bereich des Massagekopfes 42 vorgesehen. Die Montage erfolgt auf dem Zwischenring 65 und der rotierenden Achse 52 mit einem Dichtring. Oberhalb des Bodens 62 sind mehrere Antriebe 64 ersichtlich. Der Begrenzungsring 63 limitiert den Schwenkwinkel des Massagewerkzeugs 54 und schützt somit die Antriebe 64 vor Kollisionen mit dem Massagewerkzeug 54. Die Antriebe 64 können einerseits die Rotation des Massagekopfs 42 und/oder die Rotation und Verschwenkung des Massagewerkzeugs 54 bestimmen.

Bei Schlafstörungen oder zum Komfortgewinn kann eine Massageeinrichtung 38 zeitlich gesteuerte Massageprogramme zur Unterstützung des Einschlafprozesses, zur Verkürzung von Wachperioden oder als Weckfunktion durchführen. Im Fall einer Automatisierung ist die Massage jederzeit und mehrmals täglich verfügbar, was bei manuellen, von Pflegepersonen durchgeführten Massagen kaum möglich ist. Da die beschriebene Auflagevorrichtung 1 samt Spannvorrichtungen 7 und Massageeinrichtung 38 sehr geräuscharm arbeitet, muss selbst während der Nachtruhe und selbst bei Belegung in Mehrbettzimmern nicht auf eine Massage, eine lokale oder globale Kühlung oder Erwärmung oder eine Änderung der Gurtvorspannungen verzichtet werden.

Wie in Fig. 6A zu ersehen ist, kann die Auflagevorrichtung 1 auch mit einem Bediencomputer (HMI) 43 verbunden sein, der beispielsweise die Konfiguration eines Vorspannprofils oder eines Massageprogramms erlaubt. Es könnten auch die Daten der Kraftsensoren 22 aufgezeichnet, ausgewertet und beispielsweise zur Auswahl oder Optimierung der Massage herangezogen werden.

Selbstverständlich können auch andere medizinische Gerätschaften und Hilfsmittel, wie beispielsweise Detektoren oder Aufnahmevorrichtungen für eine Röntgendiagnose, als Behandlungseinrichtung 39 verwendet werden.

In Fig. 6D ist ein Massagekopf 42 für die lokale Erwärmung oder Kühlung gezeigt. Der Massagekopf 42 ist an der Unterseite 51 und über eine Drehachse 52 mit der Massageeinrichtung 38 (vgl. Fig. 6A) verbunden. Für die lokale Erwärmung ist in dem Massagekopf eine elektrische Widerstandsheizung oder Wärmelampe im Massagekopfgehäuse 66 integriert. Für eine lokale Kühlung ist das Massagekopfgehäuse 66 aus einem Material mit hoher Wärmekapazität, z.B. Kupfer, ausgeführt. Zur Kühlung wird der Massagekopf 42 in seiner Toolhalterung auf eine vorgebbare Temperatur gekühlt.

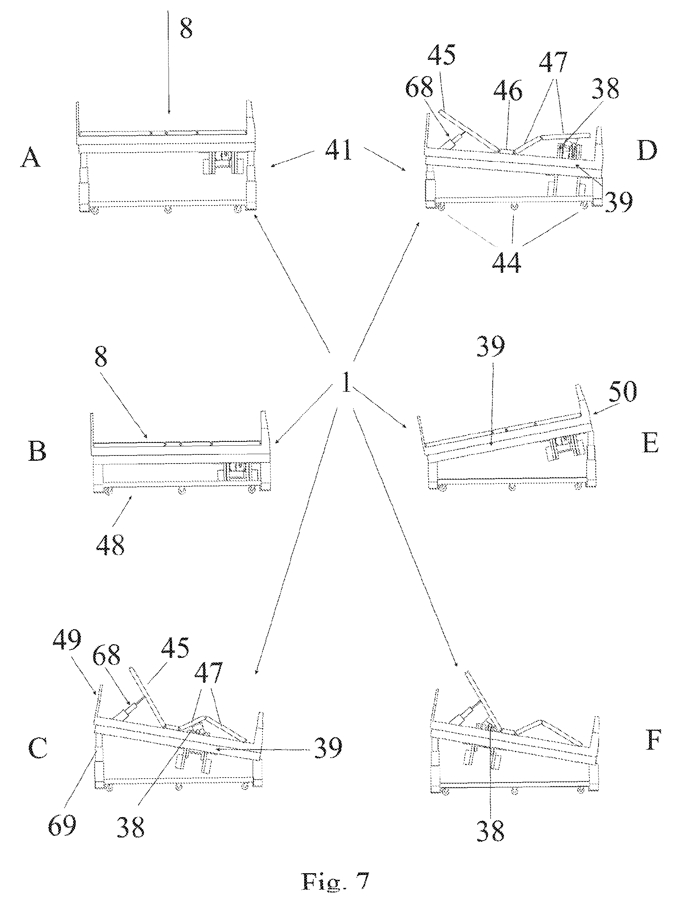

Für die Verwendung der Auflagevorrichtung 1 beispielsweise als Krankenbett können spezielle Funktionen im dritten Modul 41 - dem Unterbau - umgesetzt werden. Wie in Fig. 7 ersichtlich gehören dazu die durch Rollen 44 hergestellte Mobilität, die Fixierung durch Bremsen (nicht gezeigt), aber auch das Heben, Senken und Schrägstellen der gesamten Auflagefläche 8 oder von Teilsegmenten 45, 46, 47 der Auflagefläche 8 (vgl. Teilfiguren A bis F in Fig. 7), um die Pflegearbeit zu erleichtern und/oder als Sicherheitsmaßnahmen. Dabei kann die Behandlungseinrichtung 39, wie hier beispielhaft an der Massageeinrichtung 38 gezeigt ist, den Veränderungen der Auflagefläche 8 folgen. Die Teilfiguren zeigen im Detail:
A Eine flache Behandlungsposition für beliebige Körperstellungen, wobei die Auflagefläche 8 vorteilhaft etwa auf Hüfthöhe bzw. Arbeitshöhe des Behandlungspersonals angeordnet ist;
B Eine flache Einstiegsposition, wobei der Abstand von der Auflagefläche 8 vom Boden 48 etwa der Sitzhöhe des Benutzers entspricht, so dass ein einfaches Ein- und Aussteigen ermöglicht wird; diese Position wird auch als spezielle Schlafposition bei Gefahr des Herausfallens oder als Notfallposition (zum Wiederbeleben) gewählt;
C Eine S-förmige Sitzposition, wobei ein Rückensegment 45 etwa 45° gekippt ist (z.B. mittels eines nur schematisch eingezeichneten Druckzylinders 68), zwei Beinsegmente 47 angeordnet sind um eine abgewinkelte Beinstellung zu unterstützen und das gesamte zweite Modul 39 - z.B. mittels einer Teleskopzylindereinrichtung 69 - eine zum Kopfende 49 ansteigende Neigung aufweist. Auch diese Position wird als Notfallposition (Trendelenburg-Lagerung) verwendet. Die Positionen C und E müssen besonders schnell ausgerichtet werden können. Dabei ist die Massageeinrichtung 38 in einer Position zur Massage der Oberschenkel des Benutzers angeordnet;
D Eine leicht aufgerichtete Liegeposition mit erhöhten Rücken- und Beinsegmenten 45, 47;
E Eine flache Liegeposition, wobei das zweite Modul 39 eine zum Fußende 50 ansteigende Neigung aufweist; und
F Eine Sitzposition wie in Teilfigur D, wobei die Massageeinrichtung 38 in einer Position zur Massage des Lendenwirbelbereichs des Benutzers angeordnet ist; wobei die Positionen C und E besonders schnell ausgerichtet werden können.
Weiters kann ein beispielsweise elektrischer Hilfsantrieb vorgesehen sein, um den Ortswechsel häufig bewegter Betten zu erleichtern.

Schematisch sind wie erwähnt in Fig. 7C zwei Hubeinrichtungen 68, 69 beispielhaft gezeigt, Vergleichbare (oder andere bekannte) Hubeinrichtungen bzw. Hubsäulen können für die anderen Segmente bzw. Module eingesetzt werden, was in der Zeichnung der Einfachheit halber nicht dargestellt ist.

Auch wenn die gezeigte Auflagevorrichtung 1 insbesondere im Krankenanstaltenbereich als Krankenbett ihre Vorteile entfalten kann, kann sie aber auch im Arbeits-, Privat-, Pflege- und Wellnessbereich in Form eines Bettes oder als Stuhl, Sitz (z.B. in Verkehrsmitteln wie Auto, Bahn, Bus oder Flugzeug), als Fauteuils oder als Liege (z.B. Sonnenliege im Freibereich) eingesetzt werden. Im privaten Bereich kann die Anordnung gegebenenfalls entsprechend vereinfacht und beispielsweise auf eine Modulbauweise verzichtet werden.

## Patentansprüche

1. Auflagevorrichtung (1) für Liege- oder Sitzeinrichtungen mit einem zumindest abschnittsweise festen Rahmen (2) und mit verflochtenen, gespannten Gurten (4), die eine Auflagefläche (8) definieren und die von der oberen Auflageseite der Auflagefläche zugänglich sind, wobei zumindest einige der Gurte (4) mittels getrennt einstellbarer Spannvorrichtungen (7) mit dem Rahmen (2) verbunden sind, so dass die Vorspannung der Gurte (4) lokal einstellbar ist, **dadurch gekennzeichnet, dass** die Spannvorrichtungen (7) zwischen Gurten (4) und Rahmen (2) angeordnet und die Gurte (4) auch von der Unterseite der Auflagefläche (8) zugänglich sind, sodass die Auflagefläche (8) von der Unterseite zugänglich ist, dass die Spannvorrichtungen (7) einen Antrieb (26) zur Einstellung der Vorspannung umfassen, und dass die Antriebe (27) der Spannvorrichtungen (7) getrennt angesteuert bzw. ansteuerbar sind, so dass damit die Härte der Auflagefläche (8) jederzeit lokal anpassbar ist, wobei eine lokal einstellbare Vorspannung der Gurte (4) gemäß einem Vorspannungsprofil, z.B. automatisch, einstellbar ist.

2. Auflagevorrichtung nach Anspruch 1, **dadurch gekennzeichnet, dass** der Rahmen (2) mit den Gurten (4) ein erstes Modul (40) bildet, an dessen Unterseite eine ein zweites Modul (39) bildende Behandlungseinrichtung angeordnet ist, welche ihrerseits auf einem von einem Unterbau (41) gebildeten dritten Modul angeordnet ist.

3. Auflagevorrichtung nach Anspruch 1 oder 2, **dadurch gekennzeichnet, dass** die mit einer Spannvorrichtung (7) verbundene Gurte (4) an ihrem der Spannvorrichtung gegenüberliegenden Ende (17) fest mit dem Rahmen (2) verbunden sind.

4. Auflagevorrichtung nach Anspruch 1 oder 2, **dadurch gekennzeichnet, dass** die mit Spannvorrichtungen (7) verbundenen Gurte (4) an beiden Enden mit einer Spannvorrichtung (7) verbunden sind.

5. Auflagevorrichtung nach einem der Ansprüche 1 bis 4,
**dadurch gekennzeichnet, dass** mit jeder Spannvorrichtung (7) maximal zehn Gurte (4), bevorzugt ein einziger Gurt (4) verbunden sind.

6. Auflagevorrichtung nach einem der Ansprüche 1 bis 5, **dadurch gekennzeichnet, dass** die Spannvorrichtungen (7) eine Gleit- oder Abrollführung (18) der Gurte (4) aufweisen, wobei vorzugsweise die Gleit- oder Abrollführung (18) die Gurte (4) in Richtung zur Unterseite, insbesondere um einen Winkel von etwa 90°, umlenkt.

7. Auflagevorrichtung nach einem der Ansprüche 1 bis 6, **dadurch gekennzeichnet, dass** die Spannvorrichtungen (7) eine Federung (28) aufweisen, wobei die von der Federung (28) ausgeübte Federkraft im Wesentlichen der Vorspannung des zugehörigen Gurtes (4) entspricht, wobei vorzugsweise der Maximalhub der Federung (28), insbesondere bei einem Wert von ≤10 cm, begrenzt ist.

8. Auflagevorrichtung nach einem der Ansprüche 1 bis 7, **dadurch gekennzeichnet, dass** das vom Antrieb (26) aufgebrachte Moment, bevorzugt mindestens 1,5 Nm pro Gurt (4), ausreicht, um die Vorspannung während der Verwendung der Auflagevorrichtung (1) einzustellen.

9. Auflagevorrichtung nach einem der Ansprüche 1 bis 8, **dadurch gekennzeichnet, dass** der Antrieb (26) über eine Spindel (24) mit dem Gurt (4) verbunden ist und/oder zwischen der Federung (28) und dem Gurt (4) betriebsmäßig angeordnet ist.

10. Auflagevorrichtung nach einem der Ansprüche 1 bis 9, **dadurch gekennzeichnet, dass** die Antriebe (26) der Spannvorrichtungen (7) zeitgesteuert sind und die Vorspannung der Gurte (4) automatisch zeitabhängig veränderbar ist.

11. Auflagevorrichtung nach einem der Ansprüche 1 bis 10, **dadurch gekennzeichnet, dass** die Spannvorrichtungen (7) einen Kraftsensor (22) zur Bestimmung des Auflagedrucks umfassen, wobei der Kraftsensor (22) bevorzugt fest mit dem Gurt (4) verbunden ist, wobei vorzugsweise die Antriebe (26) der Spannvorrichtungen (7) in Abhängigkeit des vom Kraftsensor (22) bestimmten Auflagedrucks angesteuert sind.

12. Auflagevorrichtung nach einem der Ansprüche 1 bis 11, **dadurch gekennzeichnet, dass** die Zugfestigkeit der Gurte (4) und/oder die Spannvorrichtung (7) auf die, die Auflage benutzende Person abstimmbar ist und mindestens eine um einen Faktor drei höhere Zugbelastung aufweist als die maximal auftretende Gurtspannung.

13. Auflagevorrichtung nach einem der Ansprüche 2 bis 12, **dadurch gekennzeichnet, dass** die Behandlungseinrichtung (39) ein Massagegerät (38), bevorzugt ein Massagegerät (38) mit auswechselbaren oder automatisch umschaltbaren Behandlungsköpfen (42), insbesondere zur Massage oder zur Wärmebehandlung, aufweist, wobei bevorzugt das Massagegerät, z.B. mittels berührungsbehaftetem Abtasten und/oder berührungsloser, induktiver, optischer, thermischer, insbesondere kapazitiver, Messung, eine automatische Lagebestimmung des auf der Auflagefläche befindlichen Benutzers durchführt.

14. Auflagevorrichtung nach einem der Ansprüche 1 bis 13, **dadurch gekennzeichnet, dass** am Kopf- oder Fußende des Bettes (8) eine Heizung, beispielsweise eine elektrische Heizung oder ein Infrarotstrahler, und/oder eine Kühlung, beispielsweise ein Ventilator oder ein Temperierkreislauf (67) mit einem Temperiermedium, angeordnet ist.

15. Auflagevorrichtung nach einem der Ansprüche 1 bis 14 **dadurch gekennzeichnet, dass** die Gurte (4) aus einem hitze-, UV- und feuchtigkeitsbeständigen Material hergestellt sowie thermisch und chemisch desinfizierbar sind und/oder eine reibungsarme Oberfläche aufweisen, wobei der Reibungswiderstand zwischen den Gurten (4) gegenüber der Vorspannung vernachlässigbar ist.

16. Auflagevorrichtung nach einem der Ansprüche 1 bis 15, **dadurch gekennzeichnet, dass** der Rahmen (2) in Längsrichtung in bevorzugt mindestens zwei, insbesondere vier, durch Gelenke miteinander verbundene Teilsegmente (45) unterteilt ist, wobei die Gelenksachsen quer zur Längsrichtung des Rahmens angeordnet sind, wobei vorzugsweise mit dem Rahmen lineare Hilfsantriebe, insbesondere Hubsäulen, zur Einstellung der Winkellage der Teilsegmente (45) verbunden sind.

## Claims

1. A support apparatus (1) for lying or sitting equipment, comprising an at least partially fixed frame (2) and comprising intertwined tensioned straps (4) which define a support surface (8) and are accessible from the upper support side of the support surface, wherein at least some of the straps (4) are connected to the frame (2) by means of separately adjustable tensioning devices (7) such that the pretensioning of the straps (4) is locally adjustable, **characterized in that** the tensioning devices (7) are arranged between the straps (4) and the frame (2) and that the straps (4) are also accessible from the underside of the support surface (8) such that the support surface (8) is accessible from the underside, that the tensioning devices (7) comprise a drive (26) for adjusting the pretensioning, and that the drives (27) of the tensioning devices (7) are controlled and/or controllable separately such that therewith the hardness of the support surface (8) is locally adaptable at any time, wherein a locally adjustable pretensioning of the straps (4) is adjustable in accordance with a pretensioning profile, e.g. automatically.

2. The support apparatus according to claim 1, **characterized in that** the frame (2) along with the straps (4) forms a first module (40) having an equipment for treatment which forms a second module arranged at the underside thereof, said equipment for treatment in turn being arranged on a third module formed by a substructure (41).

3. The support apparatus according to claims 1 or 2, **characterized in that** the straps (4) connected to a tensioning device (7) are firmly connected to the frame (2) at their end (17) opposite to the tensioning device.

4. The support apparatus according to claims 1 or 2, **characterized in that** the straps (4) connected to tensioning devices (7) are connected to a tensioning device (7) at both ends.

5. The support apparatus according to any of claims 1 to 4, **characterized in that** a maximum of ten straps (4), preferably one single strap (4), is connected to each tensioning device (7).

6. The support apparatus according to any of claims 1 to 5, **characterized in that** the tensioning devices (7) comprise a sliding or rolling guide (18) of the straps (4), wherein preferably the sliding or rolling guide (18) deflects the straps (4) in the direction of the underside, in particular by an angle of approximately 90°.

7. The support apparatus according to any of claims 1 to 6, **characterized in that** the tensioning devices (7) comprise a resilient suspension (28), wherein the spring force exerted by the resilient suspension (28) substantially corresponds to the pretensioning of the associated strap (4), wherein preferably the maximum lift of the resilient suspension (28) is restricted, in particular at a value of ≤10 cm.

8. The support apparatus according to any of claims 1 to 7, **characterized in that** the moment applied by the drive (26), preferably at least 1.5 Nm per strap (4), is sufficient for adjusting the pretensioning during the use of the support apparatus (1).

9. The support apparatus according to any of claims 1 to 8, **characterized in that** the drive (26) is connected to the strap (4) via a leadscrew (24) and/or is operably arranged between the resilient suspension (28) and the strap (4).

10. The support apparatus according to any of claims 1 to 9, **characterized in that** the drives (26) of the tensioning devices (7) are time-controlled and that the pretensioning of the straps (4) can be modified automatically as a function of time.

11. The support apparatus according to any of claims 1 to 10, **characterized in that** the tensioning devices (7) comprise a force sensor (22) for determining the support pressure, wherein the force sensor (22) is preferably firmly connected to the strap (4), wherein preferably the drives (26) of the tensioning devices (7) are controlled as a function of the support pressure determined by the force sensor (22).

12. The support apparatus according to any of claims 1 to 11, **characterized in that** the tensile strength of the straps (4) and/or the tensioning device (7) can be adapted to the person using the support and comprises a tensile load which is at least by a factor three higher than the maximally occurring strap tensioning.

13. The support apparatus according to any of claims 2 to 12, **characterized in that** the equipment for treatment (39) comprises an equipment for massage (38), preferably an equipment for massage (38) with exchangeable or automatically switchable treatment heads (42), in particular for massage or for heat treatment, wherein preferably the equipment for massage performs an automatic determination of the position of the user being present on the support surface e.g. by means of contact-based scanning and/or contactless, inductive, optical, thermal, in particular capacitive, measurement.

14. The support apparatus according to any of claims 1 to 13, **characterized in that** a heating, for instance, an electric heating or an infrared radiator, and/or a cooling, for instance, a ventilator or a temperature control circuit (67) with a temperature control medium, is arranged at the head or foot end of the bed (8).

15. The support apparatus according to any of claims 1 to 14, **characterized in that** the straps (4) are manufactured of a heat, UV and moisture-resistant material and are thermally and chemically sanitizable and/or comprise a low-friction surface, wherein the friction resistance between the straps (4) is negligible in relation with the pretensioning.

16. The support apparatus according to any of claims 1 to 15, **characterized in that** the frame (2) is divided in longitudinal direction into preferably at least two, in particular four, partial segments (45) interconnected by hinges, wherein the hinge axes are arranged transversely to the longitudinal direction of the frame, wherein preferably linear auxiliary drives, in particular lifting columns, for adjusting the angular position of the partial segments (45) are connected to the frame.

## Revendications

1. Dispositif d'appui (1) pour des dispositifs de couchage ou d'assise avec un cadre (2) au moins partiellement fixe et avec des sangles (4) entrelacées serrées, qui définissent une surface d'appui (8) et qui sont accessibles à partir du côté supérieur d'appui de la surface d'appui, au moins certaines des sangles (4) étant reliées au moyen de dispositifs de serrage (7) réglables séparément, avec le cadre (2), de façon à ce que la précontrainte des sangles (4) soit réglable localement, **caractérisé en ce que** les dispositifs de serrage (7) sont disposés entre des sangles (4) et le cadre (2) et les sangles (4) sont également accessibles à partir du côté inférieur de la surface d'appui (8), de façon à ce que la surface d'appui (8) soit accessible à partir du côté inférieur, **en ce que** les dispositifs de serrage (7) comprennent un dispositif d'entraînement (26) pour le réglage de la précontrainte et **en ce que** les dispositifs d'entraînement (27) des dispositifs de serrage (7) sont contrôlés ou peuvent être contrôlés séparément, de façon à ce que la dureté de la surface d'appui (8) puisse être ainsi adaptée localement à tout moment, une précontrainte réglable localement des sangles (4) étant réglable selon un profil de précontrainte, par exemple automatiquement.

2. Dispositif d'appui selon la revendication 1, **caractérisé en ce que** le cadre (2) forme, avec les sangles (4), un premier module (40), sur le côté inférieur duquel se trouve un dispositif de traitement formant un deuxième module (39), qui se trouve lui-même sur un troisième module constitué d'une base (41).

3. Dispositif d'appui selon la revendication 1 ou 2, **caractérisé en ce que** les sangles (4) reliées avec un dispositif de serrage (7) sont reliées, au niveau de leur extrémité (17) opposée au dispositif de serrage, de manière solidaire avec le cadre (2).

4. Dispositif d'appui selon la revendication 1 ou 2, **caractérisé en ce que** les sangles (4) reliées avec un dispositif de serrage (7) sont reliées, aux deux extrémités, avec un dispositif de serrage (7).

5. Dispositif d'appui selon l'une des revendications 1 à 4, **caractérisé en ce que**, avec chaque dispositif de serrage (7), au maximum dix sangles (4), de préférence une seule sangle (4), sont reliées.

6. Dispositif d'appui selon l'une des revendications 1 à 5, **caractérisé en ce que** les dispositifs de serrage (7) comprennent un guidage de glissement ou de roulement (18) des sangles (4), de préférence le guidage de glissement ou de roulement (18) déviant les sangles (4) en direction du côté inférieur, plus particulièrement d'un angle d'environ 90°.

7. Dispositif d'appui selon l'une des revendications 1 à 6, **caractérisé en ce que** les dispositifs de serrage (7) comprennent une suspension (28), la force exercée par la suspension (28) correspondant globalement à la précontrainte de la sangle (4) correspondante, de préférence la course maximale de la suspension (28), plus particulièrement étant limitée à une valeur ≤ 10 cm.

8. Dispositif d'appui selon l'une des revendications 1 à 7, **caractérisé en ce que** le couple appliqué par le dispositif d'entraînement (26), de préférence d'au moins 1,5 Nm par sangle (4) suffit pour régler la précontrainte pendant l'utilisation du dispositif d'appui (1).

9. Dispositif d'appui selon l'une des revendications 1 à 8, **caractérisé en ce que** le dispositif d'entraînement (26) est relié avec la sangle (4) par l'intermédiaire d'une broche (24) et/ou est disposé entre la suspension (28) et la sangle (4) dans les conditions de fonctionnement.

10. Dispositif d'appui selon l'une des revendications 1 à 9, **caractérisé en ce que** les dispositifs d'entraînement (26) des dispositifs de serrage (7) sont contrôlés dans le temps et la précontrainte des sangles (4) peut être modifiée automatiquement en fonction du temps.

11. Dispositif d'appui selon l'une des revendications 1 à 10, **caractérisé en ce que** les dispositifs de serrage (7) comprennent un capteur de force (22) pour la détermination de la pression d'appui, le capteur de force (22) étant relié de préférence de manière solidaire avec la sangle (4), de préférence les dispositifs d'entraînement (26) des dispositifs de serrage (7) étant contrôlés en fonction de la pression d'appui déterminée par le capteur de force (22).

12. Dispositif d'appui selon l'une des revendications 1 à 11, **caractérisé en ce que** la résistance à la traction des sangles (4) et/ou le dispositif de serrage (7) peut être adapté à la personne utilisant la surface d'appui et présente une sollicitation de traction supérieure à trois fois la tension de sangle effective.

13. Dispositif d'appui selon l'une des revendications 2 à 12, **caractérisé en ce que** le dispositif de traitement (39) comprend un appareil de massage (38), de préférence un appareil de massage (38) avec des têtes de traitement (42) interchangeables ou commutables automatiquement, plus particulièrement pour le massage ou pour un traitement thermique, de préférence l'appareil de massage, effectue, par exemple au moyen d'un balayage avec contact et/ou d'une mesure sans contact, inductive, optique, thermique, plus particulièrement capacitive, une détermination automatique de la position de l'utilisateur se trouvant sur la surface d'appui.

14. Dispositif d'appui selon l'une des revendications 1 à 13, **caractérisé en ce que**, au niveau de la tête ou du pied du lit (8), est disposé un chauffage, par exemple un chauffage électrique ou un radiateur infrarouge et/ou un refroidissement, par exemple un ventilateur ou un circuit de thermostatisation (67) avec un fluide de thermostatisation.

15. Dispositif d'appui selon l'une des revendications 1 à 14, **caractérisé en ce que** les sangles (4) sont constituées d'un matériau résistant à la chaleur, aux UV et à l'humidité et peuvent être désinfectées thermiquement et chimiquement et/ou présentent une surface sans frottement, la résistance au frottement entre les sangles (4) étant négligeable par rapport à la précontrainte.

16. Dispositif d'appui selon l'une des revendications 1 à 15, **caractérisé en ce que** le cadre (2) est divisé, dans la direction longitudinale de préférence en au moins deux, plus particulièrement quatre, segments partiels (45) reliés entre eux par des articulations, les axes d'articulation étant disposés transversalement par rapport à la direction longitudinale du cadre, de préférence des entraînements auxiliaires linéaires, plus particulièrement des colonnes de levage, pour le réglage de la position angulaire des segments partiels (45), étant reliés avec le cadre.
